(19) **Europäisches Patentamt European Patent Office Office européen des brevets**

(11) **EP 4 563 229 A1**

(12) **EUROPEAN PATENT APPLICATION**
published in accordance with Art. 153(4) EPC

(43) Date of publication:
04.06.2025 Bulletin 2025/23

(51) International Patent Classification (IPC):
*B03C 5/02* (2006.01)   *C12M 1/00* (2006.01)
*G01N 37/00* (2006.01)

(21) Application number: 23845976.2

(22) Date of filing: 23.05.2023

(52) Cooperative Patent Classification (CPC):
**B01D 57/02; B01J 19/00; B03C 5/00; B03C 5/02; C12M 1/00; G01N 37/00**

(86) International application number:
**PCT/JP2023/019097**

(87) International publication number:
**WO 2024/024238 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.07.2022 JP 2022121811**

(71) Applicant: **SCREEN Holdings Co., Ltd.**
**Kyoto-shi, Kyoto 602-8585 (JP)**

(72) Inventor: **URAKAWA Satoshi**
**Kyoto-shi, Kyoto 602-8585 (JP)**

(74) Representative: **Goddar, Heinz J.**
**Boehmert & Boehmert**
**Anwaltspartnerschaft mbB**
**Pettenkoferstrasse 22**
**80336 München (DE)**

(54) **CHANNEL CHIP, DIELECTROPHORETIC DEVICE, AND CONTROL VOLTAGE CORRECTION METHOD**

(57) A channel chip (11) includes a substrate (21), a liquid receiver (FW), a pair of first electrodes (33a), (33b), and a pair of second electrodes (43a), (43b). A sample liquid or a reference liquid can be injected into the liquid receiver (FW). When a control voltage is applied, the pair of first electrodes (33a), (33b) can cause a dielectrophoretic force to act on dielectric particles (P1) contained in the sample liquid injected into the liquid receiver (FW). The pair of second electrodes (43a), (43b) are disposed at positions different from positions of the pair of first electrodes (33a), (33b). The control voltage applied between the pair of first electrodes (33a), (33b) are corrected based on the impedance between the pair of second electrodes (43a), (43b) measured through the reference liquid injected into the liquid receiver (FW).

[FIG. 1]

EP 4 563 229 A1

## Description

Technical Field

The present invention relates to a channel chip, a dielectrophoresis apparatus, and a control voltage correction method.

Background Art

Dielectrophoresis is utilized as a particle control technology in a micro space such as a microfluidic device. In the biotechnology field, various researches and developments have been conducted on characterization and separation and concentration mainly for cells or microorganisms. There are various forms, including, for example, Patent Literature 1.

The separation device described in Patent Literature 1 includes a channel through which a specimen liquid (sample liquid) containing circulating cancer cells (dielectric particles) flows in a predetermined direction, a replacement unit, an analysis unit, and a separation unit. The separation unit includes a pair of electrodes, a power supply unit, and a collection portion. The power supply unit generates an AC voltage and supplies the AC voltage between the pair of electrodes. As a result, a positive dielectrophoretic force (attractive force) acts on the cancer cells, and the cancer cells flow in the channel along the extending direction of the pair of electrodes while being attracted to the pair of electrodes and are collected by the collection portion. That is, the separation device separates cancer cells from cells such as white blood cells and collects the cancer cells.

Citation List

Patent Literature

Patent Literature 1: Japanese Patent Application Publication No. 2017-134020

Summary of Invention

Technical Problem

However, in the separation device described in Patent Literature 1, various parasitic components such as parasitic resistance and parasitic capacitance exist inside the separation unit. Therefore, the voltage value of the AC voltage actually applied to the specimen liquid is smaller than the voltage value (set value) of the AC voltage output from the power supply unit. As a result, the separation performance and collection performance of cancer cells from cells such as white blood cells may deteriorate.

The present invention has been made in view of the above problems, and an object of the present invention is to provide a channel chip, a dielectrophoresis apparatus, and a control voltage correction method that can reduce the influence of parasitic components on the separation performance and collection performance of dielectric particles contained in a sample liquid.

Solution to Problem

According to one aspect of the present invention, a channel chip includes a substrate, a liquid receiver, a pair of first electrodes, and a pair of second electrodes. The liquid receiver is disposed on one principal surface side of the substrate and into which a sample liquid or a reference liquid can be injected. The pair of first electrodes are disposed between the principal surface of the substrate and the liquid receiver and can cause a dielectrophoretic force to act on dielectric particles contained in the sample liquid injected into the liquid receiver when a control voltage is applied. The pair of second electrodes are disposed at positions different from positions of the pair of first electrodes between the principal surface of the substrate and the liquid receiver. The control voltage applied between the pair of first electrodes is corrected based on an impedance between the pair of second electrodes measured through the reference liquid injected into the liquid receiver.

In a mode of the present invention, an electric resistivity of the reference liquid is preferably smaller than an electric resistivity of the second electrode.

In a mode of the present invention, the control voltage applied between the pair of first electrodes is preferably corrected based on a correction coefficient. The correction coefficient is preferably calculated based on the impedance between the pair of second electrodes measured through the reference liquid injected into the liquid receiver and an impedance between the pair of first electrodes measured through the sample liquid injected into the liquid receiver.

In a mode of the present invention, the control voltage is preferably an AC voltage. The correction coefficient preferably has a value corresponding to a frequency of the control voltage.

In a mode of the present invention, the liquid receiver preferably includes a first channel and a second channel. It is preferable that the first channel is disposed on the principal surface side of the substrate and into which the sample liquid can be injected. It is preferable that the second channel is separated from the first channel and disposed on the principal surface side of the substrate and into which the reference liquid can be injected. The pair of first electrodes are preferably disposed between the principal surface of the substrate and the first channel. Preferably, the pair of second electrodes are separated from the first channel and the pair of first electrodes and disposed between the principal surface of the substrate and the second channel. The control voltage applied between the pair of first electrodes is preferably corrected based on the impedance between the pair of second electrodes measured through the reference liquid injected into the second channel.

In a mode of the present invention, the liquid receiver preferably includes a first channel and a retention portion. It is preferable that the first channel is disposed on the principal surface side of the substrate and into which the sample liquid can be injected. It is preferable that the retention portion is separated from the first channel and disposed on the principal surface side of the substrate and into which the reference liquid can be injected. The pair of first electrodes are preferably disposed between the principal surface of the substrate and the first channel. It is preferable that the pair of second electrodes are separated from the first channel and the pair of first electrodes and disposed between the principal surface of the substrate and the retention portion. The control voltage applied between the pair of first electrodes is preferably corrected based on the impedance between the pair of second electrodes measured through the reference liquid injected into the retention portion.

In a mode of the present invention, the liquid receiver preferably includes a first channel. It is preferable that the pair of first electrodes are disposed between the principal surface of the substrate and the first channel and can cause the dielectrophoretic force to act on the dielectric particles contained in the sample liquid injected into the first channel when the control voltage is applied in a state where the reference liquid is not injected into the first channel. The pair of second electrodes are preferably disposed at positions different from positions of the pair of first electrodes between the principal surface of the substrate and the first channel. The control voltage applied between the pair of first electrodes is preferably corrected based on the impedance between the pair of second electrodes measured through the reference liquid injected into the first channel in a state where the sample liquid is not injected into the first channel.

According to another aspect of the present invention, a dielectrophoresis apparatus includes a channel chip and a voltage controller. The channel chip includes a substrate, a liquid receiver, a pair of first electrodes, and a pair of second electrodes. The liquid receiver is disposed on one principal surface side of the substrate and into which a sample liquid or a reference liquid can be injected. The pair of first electrodes are disposed between the principal surface of the substrate and the liquid receiver and can cause a dielectrophoretic force to act on dielectric particles contained in the sample liquid injected into the liquid receiver when a control voltage is applied. The pair of second electrodes are disposed at positions different from positions of the pair of first electrodes between the principal surface of the substrate and the liquid receiver. The voltage controller corrects the control voltage applied between the pair of first electrodes based on an impedance between the pair of second electrodes measured through the reference liquid injected into the liquid receiver.

According to still another aspect of the present invention, a control voltage correction method uses a channel chip. The channel chip includes a substrate, a liquid receiver, a pair of first electrodes, and a pair of second electrodes. The liquid receiver is disposed on one principal surface side of the substrate and into which a sample liquid or a reference liquid can be injected. The pair of first electrodes are disposed between the principal surface of the substrate and the liquid receiver and can cause a dielectrophoretic force to act on dielectric particles contained in the sample liquid injected into the liquid receiver when a control voltage is applied. The pair of second electrodes are disposed at positions different from positions of the pair of first electrodes between the principal surface of the substrate and the liquid receiver. The control voltage correction method includes a step of measuring an impedance between the pair of second electrodes through the reference liquid injected into the liquid receiver and a step of correcting the control voltage applied between the pair of first electrodes based on the impedance between the pair of second electrodes.

In a mode of the present invention, in the control voltage correction method, the electric resistivity of the reference liquid is preferably smaller than the electric resistivity of the second electrode.

In a mode of the present invention, the control voltage correction method preferably further includes a step of measuring an impedance between the pair of first electrodes through the sample liquid injected into the liquid receiver. The step of correcting the control voltage preferably includes a step of calculating a correction coefficient based on the impedance between the pair of second electrodes measured through the reference liquid injected into the liquid receiver and the impedance between the pair of first electrodes measured through the sample liquid injected into the liquid receiver and a step of correcting the control voltage applied between the pair of first electrodes based on the correction coefficient.

In a mode of the present invention, the control voltage is preferably an AC voltage. The step of calculating the correction coefficient and the step of correcting the control voltage are preferably executed each time a frequency of the control voltage is changed.

Advantageous Effects of Invention

According to the present invention, it is possible to reduce the influence of parasitic components on the separation performance and the collection performance of dielectric particles contained in a sample liquid.

Brief Description of Drawings

[FIG. 1] is a plan view illustrating a dielectrophoresis apparatus according to a preferred embodiment of the present invention.
[FIG. 2] is an enlarged view of a first electrode and a second electrode illustrated in FIG. 1.

[FIG. 3] is a schematic cross-sectional view of a channel chip taken along line III-III in FIG. 2.

[FIG. 4] is a schematic cross-sectional view of the channel chip taken along line IV-IV in FIG. 2.

[FIG. 5] is a detailed cross-sectional view of the channel chip taken along line V-V in FIG. 2.

[FIG. 6] is a flowchart illustrating a control voltage correction method according to the present preferred embodiment.

[FIG. 7] is a circuit diagram of an equivalent circuit illustrating parasitic components of a channel chip according to the present preferred embodiment.

[FIG. 8] is a graph illustrating the impedance measured through the sample liquid and the impedance measured through the reference liquid according to the present preferred embodiment.

[FIG. 9] is a graph illustrating the difference value between the impedance measured through the sample liquid and the impedance measured through the reference liquid according to the present preferred embodiment.

[FIG. 10] is a graph illustrating the frequency dependence of voltage output efficiency according to the present preferred embodiment.

[FIG. 11] is a graph illustrating an example of the voltage value of a voltage actually applied to the sample liquid when a control voltage having a specific voltage value is applied to the first electrode according to the present preferred embodiment.

[FIG. 12] is a graph illustrating an example of voltage output efficiency according to the present preferred embodiment.

[FIG. 13] is a graph illustrating an example of a correction coefficient according to the present preferred embodiment.

[FIG. 14] is a graph illustrating the voltage value of a voltage actually applied to the sample liquid when a corrected control voltage is applied to the first electrode according to the present preferred embodiment.

[FIG. 15] is a plan view illustrating a retention portion and a second electrode of a channel chip according to a first modified example of the present preferred embodiment.

[FIG. 16] is a schematic cross-sectional view of the channel chip taken along line XVI-XVI in FIG. 15.

[FIG. 17] is a plan view illustrating a dielectrophoresis apparatus according to a second modified example of the present preferred embodiment.

Description of Embodiments

Hereinafter, a preferred embodiment of the present invention will be described with reference to the drawings. It is noted that in the drawings, the same or corresponding portions will be given the same reference signs and will not be repeatedly described.

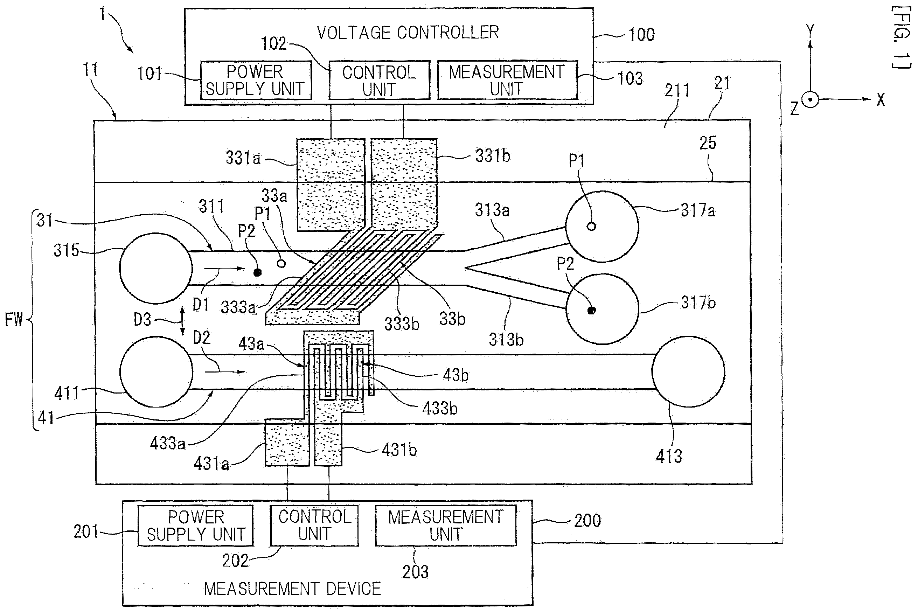

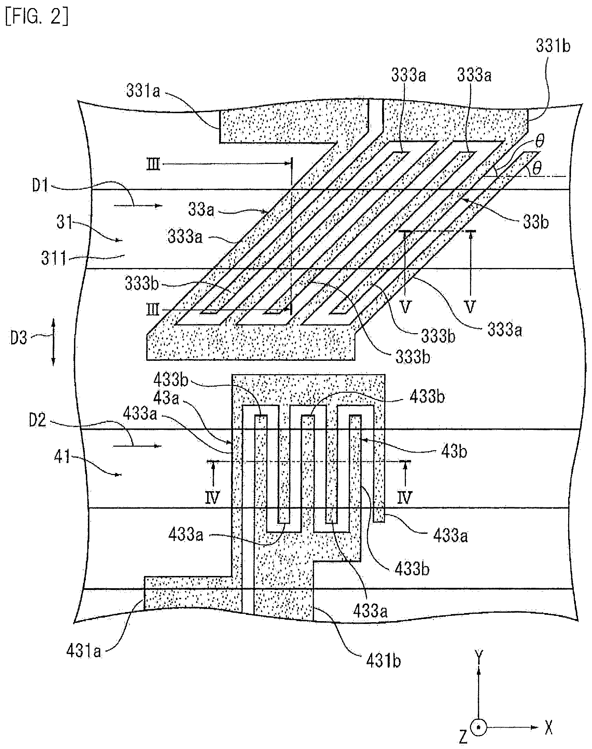

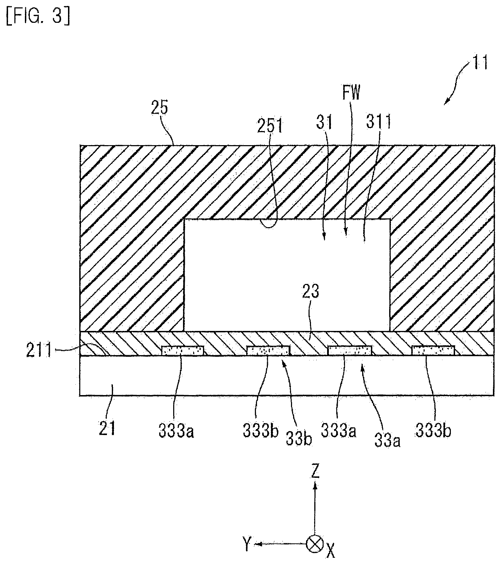

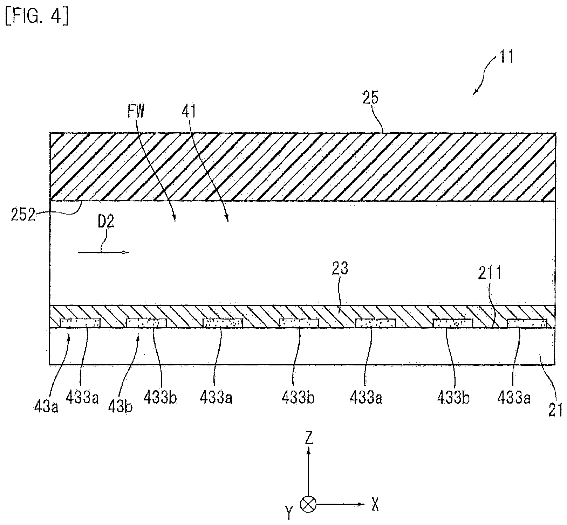

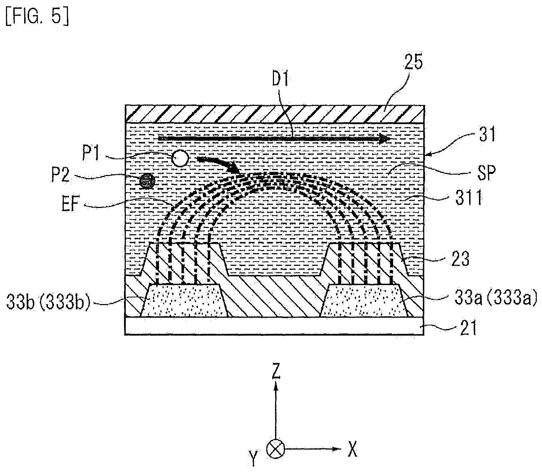

A dielectrophoresis apparatus 1 according to a preferred embodiment of the present invention will be described with reference to FIGS. 1 to 12. First, the dielectrophoresis apparatus 1 will be described with reference to FIGS. 1 to 5. FIG. 1 is a plan view illustrating the dielectrophoresis apparatus 1. FIG. 2 is an enlarged view of first electrodes 33a and 33b and second electrodes 43a and 43b illustrated in FIG. 1. FIG. 3 is a schematic cross-sectional view taken along line III-III in FIG. 2. FIG. 4 is a schematic cross-sectional view taken along line IV-IV in FIG. 2. FIG. 5 is a detailed cross-sectional view taken along line V-V in FIG. 2.

As illustrated in FIG. 1, the dielectrophoresis apparatus 1 causes a dielectrophoretic force to act on dielectric particles P1 contained in the sample liquid (specimen liquid), thereby separating the dielectric particles P1 from other components P2 and collecting the dielectric particles P1. The sample liquid is, for example, a body fluid such as blood. Blood is an example of a suspension. The dielectric particles P1 are, for example, cells, proteins, nucleic acids, or microorganisms. The cells are, for example, cancer cells. The other components P2 are, for example, a type of dielectric particle different from a specific type of the dielectric particle P1. As an example, the sample liquid is blood, the dielectric particles P1 are cancer cells, and the other components P2 are white blood cells.

Specifically, the dielectrophoresis apparatus 1 includes a channel chip 11. The channel chip 11 causes a dielectrophoretic force to act on the dielectric particles P1 contained in the sample liquid, thereby separating the dielectric particles P1 from the other components P2 and collecting the dielectric particles P1. The diameter of the dielectric particle P1 is, for example, several $\mu$m or more and tens $\mu$m or less.

The channel chip 11 includes a substrate 21, a liquid receiver FW, a pair of first electrodes 33a and 33b, a pair of second electrodes 43a and 43b, a pair of first electrode pad portions 331a and 331b, and a pair of second electrode pad portions 431a and 431b.

The material of the substrate 21 is, for example, quartz glass. However, the material of the substrate 21 is not limited to quartz glass. The substrate 21 has a substantially rectangular flat plate shape. However, the shape of the substrate 21 is not limited to the rectangular flat plate shape.

The liquid receiver FW is disposed on a side of one principal surface 211 of the pair of principal surfaces of the substrate 21. In the example of FIG. 1, the principal surface 211 on one side of the substrate 21 is the upper surface of the substrate 21, and a principal surface on the other side of the substrate 21 is the lower surface of the substrate 21.

A sample liquid or a reference liquid can be injected into the liquid receiver FW. The liquid receiver FW receives the sample liquid or the reference liquid. The reference liquid is a liquid to be referred to when a parasitic component other than the parasitic component in the sample liquid among the parasitic components in the channel chip 11 is extracted. The parasitic component is one or more components of parasitic resistance, parasitic capacitance, and parasitic inductance. Details of the parasitic component will be described later.

The electric resistance of the reference liquid is smaller than the electric resistance of the second electrodes 43a and 43b. Specifically, the electric resistivity of the reference liquid is smaller than the electric resistivity of the second electrodes 43a and 43b. Also, the electric resistance of the reference liquid is smaller than the electric resistance of the sample liquid. Specifically, the electric resistivity of the reference liquid is smaller than the electric resistivity of the sample liquid. The reference liquid is, for example, an aqueous solution of sodium chloride (NaCl) or an aqueous solution of potassium chloride (KCl). The conductivity of the reference liquid is, for example, 100 $\mu$S/cm or more.

The liquid receiver FW includes a first channel 31. A sample liquid can be injected into the first channel 31. The sample liquid flows through the first channel 31. The first channel 31 includes a separation channel 311 and a pair of collection channels 313a and 313b. The channel chip 11 further includes a first supply portion 315, a first collection portion 317a, and a second collection portion 317b.

The separation channel 311 extends linearly along a direction D1. One end of the separation channel 311 is connected to the first supply portion 315. The first supply portion 315 supplies the sample liquid to the separation channel 311. The first supply portion 315 has, for example, an opening. The first supply portion 315 is connected to, for example, a supply source of the sample liquid through a tube.

The first channel 31 branches into the collection channel 313a and the collection channel 313b at the other end of the separation channel 311.

The collection channel 313a is a channel into which the dielectric particles P1 from the separation channel 311 flow. The collection channel 313b is a channel into which the other components P2 from the separation channel 311 flow. One end of the collection channel 313a and one end of the collection channel 313b are connected to the other end of the separation channel 311. The other end of the collection channel 313a is connected to the first collection portion 317a. The other end of the collection channel 313b is connected to the second collection portion 317b. The first collection portion 317a collects the dielectric particles P1 to be separated. The first collection portion 317a may have, for example, an opening and supply a solution containing the dielectric particles P1 from the channel chip 11 to the outside. The second collection portion 317b collects the other components P2 that are non-separation targets. The second collection portion 317b may have, for example, an opening and supply a solution containing the other components P2 from the channel chip 11 to the outside.

When a control voltage Vcn is applied, the first electrodes 33a and 33b can cause a dielectrophoretic force to act on the dielectric particles P1 contained in the sample liquid injected into the liquid receiver FW. In the example of FIG. 1, when the control voltage Vcn is applied, the first electrodes 33a and 33b can cause a dielectrophoretic force to act on the dielectric particles P1 contained in the sample liquid injected into the first channel 31. The material of the first electrodes 33a and 33b is metal such as aluminum.

The first electrodes 33a and 33b can apply a measurement voltage Vm1 to the sample liquid injected into the liquid receiver FW. The measurement voltage Vm1 is an AC voltage. The measurement voltage Vm1 is a voltage for measuring an impedance IM1 between the first electrodes 33a and 33b. In the example of FIG. 1, the first electrodes 33a and 33b apply the measurement voltage Vm1 to the sample liquid injected into the first channel 31.

As illustrated in FIG. 2, the first electrodes 33a and 33b intersect the first channel 31 (the separation channel 311) at an acute angle θ in plan view. Each of the first electrodes 33a and 33b is a comb-shaped electrode. The first electrode 33a and the first electrode 33b are electrically separated from each other.

The first electrode 33a includes a plurality of first line portions 333a. The plurality of first line portions 333a are arranged at equal intervals in the direction D1. The direction D1 indicates a direction in which the first channel 31 (the separation channel 311) extends. The plurality of first line portions 333a intersect with the first channel 31 (the separation channel 311) at the acute angle θ in plan view. That is, the plurality of first line portions 333a are inclined at the acute angle θ with respect to the direction D1 in plan view. The inclination angle θ of the first line portion 333a with respect to the direction D1 is, for example, 10° or more and 60° or less. One end of one first line portion 333a of the plurality of first line portions 333a is connected to the first electrode pad portion 331a. The other ends of the plurality of first line portions 333a are connected. The first line portion 333a extends at the acute angle θ with respect to the direction D1 between one side and the other side in a direction D3. The direction D3 is substantially orthogonal to the direction D1 along the substrate 21.

The first electrode 33b includes a plurality of first line portions 333b. The plurality of first line portions 333b are arranged at equal intervals in the direction D1. The plurality of first line portions 333b intersect the first channel 31 (the separation channel 311) at the acute angle θ in plan view. That is, the plurality of first line portions 333b are inclined at the acute angle θ with respect to the direction D1 in plan view. The inclination angle θ of the first line portion 333b with respect to the direction D1 is, for example, 10° or more and 60° or less. One ends of the plurality of first line portions 333b are connected to the first electrode pad portion 331b. The first line portion 333b extends at the acute angle θ with respect to the direction D1 between one side and the other side in the direction D3.

The first line portions 333a and the first line portions 333b are alternately arranged at intervals in the direction D1. The inclination angle θ of the first line portion 333a and the inclination angle θ of the first line portion 333b are substantially the same.

As illustrated in FIGS. 2 and 3, the first electrodes 33a and 33b are disposed between the principal surface 211 of the substrate 21 and the liquid receiver FW (FIG. 1). In the example of FIG. 3, the first electrodes 33a and 33b are disposed between principal surface 211 of substrate 21 and first channel 31 (the separation channel 311). Specifically, the first line portions 333a and 333b are disposed between the principal surface 211 of the substrate 21 and the first channel 31 (the separation channel 311).

As illustrated in FIG. 3, the first electrodes 33a and 33b are disposed on the principal surface 211 of the substrate 21. The channel chip 11 further includes an insulating protective film 23 and a channel cover 25. The insulating protective film 23 covers the first electrodes 33a and 33b. The insulating protective film 23 is, for example, an oxide film. The oxide film is, for example, a silicon oxide film (SiOx). Note that the insulating protective film 23 is not particularly limited as long as it is an insulator and may be, for example, a nitride film. The nitride film is, for example, a silicon nitride film. Since the first electrodes 33a and 33b are covered with the insulating protective film 23, an electrochemical reaction occurring at the interface between the electrode metal and the liquid can be suppressed, and deterioration and wear of the electrode metal over time can be suppressed.

A channel cover 25 is disposed on an upper surface of the insulating protective film 23. The material of the channel cover 25 is, for example, silicone such as dimethylpolysiloxane (PDMS). The channel cover 25 has a recessed channel groove 251. The channel groove 251 extends along the direction D1 (FIG. 2). The width of the channel groove 251 in the direction D3 is, for example, a microscale quantity. The channel groove 251 and the upper surface of the insulating protective film 23 constitute the first channel 31. By attaching the channel cover 25 to a side of the principal surface 211 of the substrate 2 in this manner, the first channel 31 is disposed on the side of the principal surface 211 of the substrate 21.

Returning to FIG. 1, the width of the substrate 21 in the direction D3 is larger than the width of the channel cover 25 in the direction D3. The first electrode pad portions 331a and 331b are disposed away from the first channel 31 (the separation channel 311) on one side of the first channel 31 in the direction D3. At least a part of the first electrode pad portion 331a and at least a part of the first electrode pad portion 331b are disposed outside the channel cover 25. Further, the portions of the first electrode pad portions 331a and 331b which are disposed outside the channel cover 25 are not covered with the insulating protective film 23. Therefore, the first electrode pad portions 331a and 331b can be electrically connected to a voltage controller 100 outside the channel cover 25.

The first electrode pad portion 331a is connected to the first electrode 33a. The first electrode pad portion 331b is connected to the first electrode 33b.

The liquid receiver FW further includes a second channel 41. A reference liquid can be injected into the second channel 41. The second channel 41 extends linearly along a direction D2. The direction D2 is substantially parallel to the direction D1. The direction D2 indicates a direction in which the second channel 41 extends. The second channel 41 is separated from the first channel 31 in the direction D3. The direction D3 is substantially orthogonal to the directions D1 and D2 along the principal surface 211 of the substrate 21. The reference liquid flows through the second channel 41. The channel chip 11 further includes a second supply portion 411 and a recovery portion 413.

One end of the second channel 41 is connected to the second supply portion 411. The second supply portion 411 supplies the reference liquid to the second channel 41. The second supply portion 411 has, for example, an opening. The second supply portion 411 is connected to the supply source of the reference liquid through, for example, a tube. The other end of the second channel 41 is connected to the recovery portion 413. The recovery portion 413 collects the reference liquid flowing from the second channel 41. The recovery portion 413 may have, for example, an opening and supply the reference liquid from the channel chip 11 to the outside.

The second electrodes 43a and 43b apply a measurement voltage Vm2 to the reference liquid injected into the liquid receiver FW. The measurement voltage Vm2 is an AC voltage. The measurement voltage Vm2 is a voltage for measuring an impedance IM2 between the second electrodes 43a and 43b. In the example of FIG. 1, the second electrodes 43a and 43b apply the measurement voltage Vm2 to the reference liquid injected into the second channel 41. The material of the second electrodes 43a and 43b is a metal such as aluminum.

As illustrated in FIG. 2, the second electrodes 43a and 43b are arranged adjacent to the first electrodes 33a and 33b while being separated from the first electrodes 33a and 33b and the first channel 31 in the direction D3. The second electrodes 43a and 43b are substantially orthogonal to the second channel 41 in plan view. Each of the second electrodes 43a and 43b is a comb-shaped electrode. The second electrodes 43a and 43b are electrically separated from each other.

The second electrode 43a includes a plurality of second line portions 433a. The plurality of second line portions 433a are arranged at equal intervals in the direction D2. The plurality of second line portions 433a are substantially orthogonal to the second channel 41 in plan view. One ends of the plurality of second line portions 433a are connected. The other end of one second line portion 433a of the plurality of second line portions 433a is connected to the second electrode pad portion 431a. The second line portion 433a extends between one side and the other side in the direction D3 such as to be substantially orthogonal to the direction D1.

The second electrode 43b includes a plurality of second line portions 433b. The plurality of second line portions 433b are arranged at equal intervals in the direction D2. The plurality of second line portions 433b are substantially orthogonal to the second channel 41 in plan view. That is, the plurality of second line portions 433b are substantially orthogonal to the direction D2 in plan view. The other end of one end and the other end of the plurality of second line portions 433b is connected to the second electrode pad portion 431b. The second line portion 433b extends between one side and the other side in the direction D3 such as to be substantially orthogonal to the direction D1.

The second line portions 433a and the second line portions 433b are alternately arranged at intervals in the direction D2.

As illustrated in FIGS. 2 and 4, the second electrodes 43a and 43b are disposed at positions different from positions of the first electrodes 33a and 33b between the principal surface 211 of the substrate 21 and the liquid receiver FW (FIG. 1). In the example of FIG. 4, the second electrodes 43a and 43b are disposed at positions different from positions of the first electrodes 33a and 33b between the principal surface 211 of the substrate 21 and the second channel 41. Specifically, the second line portions 433a and 433b are disposed between the principal surface 211 of the substrate 21 and the second channel 41.

As illustrated in FIG. 4, the second electrodes 43a and 43b are disposed on the principal surface 211 of the substrate 21. The insulating protective film 23 covers the second electrodes 43a and 43b. Since the second electrodes 43a and 43b are covered with the insulating protective film 23, an electrochemical reaction occurring at the interface between the electrode metal and the liquid can be suppressed, and deterioration and wear of the electrode metal over time can be suppressed.

A channel cover 25 is disposed on an upper surface of the insulating protective film 23. The channel cover 25 has a recessed channel groove 252 similar to the channel groove 251 (FIG. 3). The channel groove 252 extends along the direction D2. The width of the channel groove 252 in the direction D3 (FIG. 2) is, for example, a microscale quantity. The channel groove 252 and the upper surface of the insulating protective film 23 constitute the second channel 41. As described above, by attaching the channel cover 25 to a side of the principal surface 211 of the substrate 2, the second channel 41 is disposed on a side of the principal surface 211 of the substrate 21.

Returning to FIG. 1, the second electrode pad portions 431a and 431b are disposed away from the second channel 41 on the other side of one side and the other side of the second channel 41 in the direction D3. At least a part of the second electrode pad portion 431a and at least a part of the second electrode pad portion 431b are disposed outside the channel cover 25. Also, portions of the second electrode pad portions 431a and 431b which are disposed outside the channel cover 25 are not covered with the insulating protective film 23. Therefore, the second electrode pad portions 431a and 431b can be electrically connected to a measurement device 200 outside the channel cover 25.

The second electrode pad portion 431a is connected to the second electrode 43a. The second electrode pad portion 431b is connected to the second electrode 43b.

The dielectrophoresis apparatus 1 further includes the voltage controller 100. The voltage controller 100 is electrically connected to the first electrode pad portions 331a and 331b.

The voltage controller 100 applies the control voltage Vcn corresponding to the dielectric particles P1 to the first electrodes 33a and 33b through the first electrode pad portions 331a and 331b. That is, the voltage controller 100 applies the control voltage Vcn corresponding to the dielectric particles P1 between the first electrode 33a and the first electrode 33b through the first electrode pad portions 331a and 331b. The control voltage Vcn is an AC voltage. The control voltage Vcn corresponding to the dielectric particles P1 is a voltage that has a frequency that generates an electric field that specifically applies a dielectrophoretic force (attractive force) to the dielectric particles P1 and has a magnitude that does not destroy the dielectric particles P1.

Specifically, the frequency of the control voltage Vcn is set such that a positive dielectrophoretic force (attractive force) acts on the dielectric particles P1 by the first electrodes 33a and 33b. Therefore, a positive dielectrophoretic force (attractive force) acts on the dielectric particles P1, and the dielectric particles P1 flow in the separation channel 311 along the extending direction of the first electrodes 33a and 33b (the first line portions 333a and 333b) while being attracted to the first electrodes 33a and 33b (the first line portions 333a and 333b) and are collected to the first collection portion 317a through the collection channel 313a. On the other hand, the frequency of the control voltage Vcn is set such that no dielectrophoretic force acts on the other components P2, or even if a positive or negative dielectrophoretic force (attractive force or repulsive force) acts on the other components P2, the dielectrophoretic force becomes relatively small. Therefore, the other components P2 are caused to flow by the flow in the direction D1 in the separation channel 311 and are collected by the second collection portion 317b through the collection channel 313b.

As described above with reference to FIG. 1, by causing a positive dielectrophoretic force (attractive force) to act on the dielectric particles P1, the dielectric particles P1 are separated from the other components P2 and are collected in the first collection portion 317a.

Next, the principle of separating the dielectric particles P1 from the other components P2 will be described with reference to FIG. 5. FIG. 5 is a detailed cross-sectional view taken along line V-V in FIG. 2. As illustrated in FIG. 5, the separation of the dielectric particles P1 from the other components P2 is performed through the capture of the dielectric particles P1. In this case, the capturing force with respect to the dielectric particles P1 is determined by the balance between the traveling force of a sample liquid SP in the direction D1 and the electric attractive force caused by an electric field EF generated by the pair of first electrodes 33a and 33b. The direction D1 indicates the flow direction of the sample liquid SP in the first channel 31 (the separation channel 311). Also, the inclination angle $\theta$ (FIG. 2) of the first electrodes 33a and 33b also contributes to the capture and separation of the dielectric particles P1. Hereinafter, in the present preferred embodiment, attention is paid to the electric attractive force (electric restraint force) regarding the capture and separation of the dielectric particles P1. Specifically, in the present preferred embodiment, attention is paid to the control voltage Vcn applied between the first electrodes 33a and 33b, and the attenuation of the control voltage Vcn caused by a parasitic component in the channel chip 11 is suppressed. Details will be described later.

Returning to FIG. 1, the voltage controller 100 can also apply the measurement voltage Vm1 to the first electrodes 33a and 33b through the first electrode pad portions 331a and 331b and measure the impedance IM1 between the first electrodes 33a and 33b through the sample liquid. In this case, the voltage controller 100 measures the impedance IM1 for each frequency while changing the frequency of the measurement voltage Vm1.

The voltage controller 100 includes a power supply unit 101, a control unit 102, and a measurement unit 103.

The control unit 102 controls the power supply unit 101 and the measurement unit 103. The control unit 102 includes, for example, a processor and a storage device. The processor is, for example, a central processing unit (CPU) or a micro processing unit (MPU). The storage device stores data and a computer program. The storage device includes a main storage device such as a semiconductor memory and an auxiliary storage device such as a semiconductor memory, a solid state drive, and/or a hard disk drive. The storage device may include removable media. The storage device corresponds to an example of a non-transitory computer readable storage medium.

The power supply unit 101 generates the control voltage Vcn or the measurement voltage Vm1 corresponding to the dielectric particles P1 to be separated. Then, the power supply unit 101 applies the control voltage Vcn or the measurement voltage Vm1 to the first electrodes 33a and 33b through the first electrode pad portions 331a and 331b. The power supply unit 101 is, for example, a signal generator such as a function generator.

In a state where the measurement voltage Vm1 is applied between the first electrodes 33a and 33b by the power supply unit 101, the measurement unit 103 measures the impedance IM1 between the pair of first electrodes 33a and 33b through the sample liquid injected into the liquid receiver FW. In the example of FIG. 1, the measurement unit 103 measures the impedance IM1 between the pair of first electrodes 33a and 33b through the sample liquid injected into the first channel 31. Specifically, the measurement unit 103 measures a current flowing between the first electrodes 33a and 33b through the first electrodes 33a and 33b in a state where the measurement voltage Vm1 is applied between the first electrodes 33a and 33b by the power supply unit 101. The measurement unit 103 calculates the impedance IM1 between the first electrodes 33a and 33b from the measurement voltage Vm1 and the current measured through the first electrodes 33a and 33b. The measurement unit 103 is, for example, a measurement instrument such as an oscilloscope.

The voltage controller 100 may include, for example, a source measure unit.

The dielectrophoresis apparatus 1 further includes the measurement device 200. The measurement device 200 is electrically connected to the second electrode pad portions 431a and 431b.

The measurement device 200 applies the measurement voltage Vm2 to the second electrodes 43a and 43b through the second electrode pad portions 431a and 431b and measures the impedance IM2 between the second electrodes 43a and 43b through the reference liquid. In this case, the measurement device 200 measures the impedance IM2 for each frequency while changing the frequency of the measurement voltage Vm2.

The measurement device 200 includes a power supply unit 201, a control unit 202, and a measurement unit 203.

The control unit 202 controls the power supply unit 201 and the measurement unit 203. The configuration of the control unit 202 is similar to the configuration of the control unit 102.

The power supply unit 201 generates the measurement voltage Vm2. Then, the power supply unit 201 applies the measurement voltage Vm2 to the second electrodes 43a and 43b through the second electrode pad portions 431a and 431b. The power supply unit 201 is, for example, a signal generator such as a function generator.

In a state where the measurement voltage Vm2 is applied between the second electrodes 43a and 43b by the power supply unit 201, the measurement unit 203 measures the impedance IM2 between the pair of second electrodes 43a and 43b through the reference liquid injected into the liquid receiver FW. In the example of FIG. 1, the measurement unit 203 measures the impedance IM2 between the pair of second electrodes 43a and 43b through the reference liquid injected into the second channel 41. Specifically, the measurement unit 203 measures a current flowing between the second electrodes 43a and 43b through the second electrodes 43a and 43b in a state where the measurement voltage Vm2 is applied between the second electrodes 43a and 43b by the power supply unit 201. The measurement unit 203 calculates the impedance IM2 between the second electrodes 43a and 43b from the measurement voltage Vm2 and the current measured through the second electrodes 43a and 43b. The measurement unit 203 is, for example, a measurement instrument such as an oscilloscope.

The measurement unit 203 outputs data indicating the frequency of the measurement voltage Vm2 and the impedance IM2 between the second electrodes 43a and 43b to the control unit 102 of the voltage controller 100.

The measurement device 200 may include, for example, a source measure unit.

As described above with reference to FIG. 1, according to the present preferred embodiment, the first channel 31 into which the sample liquid is injected and the second channel 41 into which the reference liquid is injected are separated from each other. Then, the impedance IM1 of the sample liquid and the impedance IM2 of the reference liquid are separately measured. Therefore, the first channel 31 can be prevented from being contaminated with the reference liquid, and the second channel 41 can be prevented from being contaminated with the sample liquid. Also, the measurement of the impedance IM1 and the measurement of the impedance IM2 can be executed in parallel. As a result, the throughput of the separation and collection processing of the dielectric particles P1 can be improved.

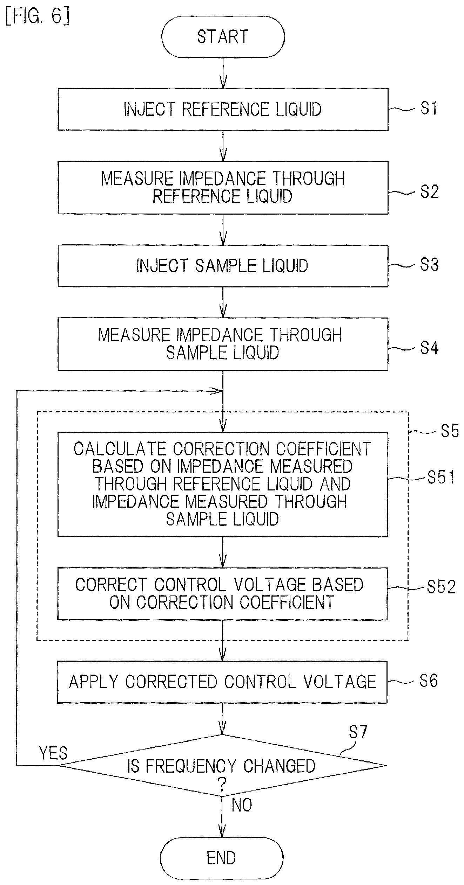

Next, a control voltage correction method according to the present preferred embodiment will be described with reference to FIGS. 1 and 6. In the control voltage correction method, the channel chip 11 is used. The control voltage correction method is executed by the dielectrophoresis apparatus 1. In the present preferred embodiment, by executing the control voltage correction method, it is possible to reduce the influence of the parasitic component in the channel chip 11 on the separation performance and the collection performance of the dielectric particles P1 from the other components P2 in the channel chip 11.

FIG. 6 is a flowchart illustrating a control voltage correction method according to the present preferred embodiment. As illustrated in FIG. 6, the control voltage correction method includes steps S1 to S7.

As illustrated in FIGS. 1 and 6, first, in step S1, a reference liquid is injected into the liquid receiver FW. In the present preferred embodiment, in step S1, the second supply portion 411 injects the reference liquid into the second channel 41.

Next, in step S2, the power supply unit 201 of the measurement device 200 applies the measurement voltage Vm2 between the pair of second electrodes 43a and 43b, and the measurement unit 203 of the measurement device 200 measures the impedance IM2 between the pair of second electrodes 43a and 43b through the reference liquid injected into the liquid receiver FW. In the present preferred embodiment, in step S2, the measurement unit 203 measures the impedance IM2 between the pair of second electrodes 43a and 43b through the reference liquid injected into the second channel 41. The measurement voltage Vm2 has a specific voltage value Vx. The measurement voltage Vm2 is an AC voltage. Therefore, the specific voltage value Vx is indicated by an effective value.

Preferably, the measurement unit 203 measures the impedance IM2 between the pair of second electrodes 43a and 43b through the reference liquid for each frequency while changing the frequency of the measurement voltage Vm2. Then, the storage device of the control unit 202 stores the impedance IM2 between the pair of second electrodes 43a and 43b for each frequency of the measurement voltage Vm2. The control unit 202 outputs data indicating the impedance IM2 for each frequency to the voltage controller 100. Then, in the voltage controller 100, the storage device of the control unit 102 stores the impedance IM2 for each frequency of the measurement voltage Vm2.

Next, in step S3, the sample liquid is injected into the liquid receiver FW. In the present preferred embodiment, in step S3, the first supply portion 315 injects the sample liquid into the first channel 31 (the separation channel 311).

Next, in step S4, the power supply unit 101 of the voltage controller 100 applies the measurement voltage Vm1 between the pair of first electrodes 33a and 33b, and the measurement unit 103 of the voltage controller 100 measures the impedance IM1 between the pair of first electrodes 33a and 33b through the sample liquid injected into the liquid receiver FW. In the present preferred embodiment, in step S4, the measurement unit 103 measures the impedance IM1 between the pair of first electrodes 33a and 33b through the sample liquid injected into the first channel 31 (the separation channel 311). The measurement voltage Vm1 is an AC voltage. The measurement voltage Vm1 has the same specific voltage value Vx as the measurement voltage Vm2.

Preferably, the measurement unit 103 measures the impedance IM1 between the pair of first electrodes 33a and 33b through the sample liquid for each frequency while changing the frequency of the measurement voltage Vm1. Then, the storage device of the control unit 102 stores the impedance IM1 between the pair of first electrodes 33a and 33b for each frequency of the measurement voltage Vm1.

Next, in step S5, the control unit 102 of the voltage controller 100 corrects the control voltage Vcn applied between the pair of first electrodes 33a and 33b based on the impedance IM2 between the pair of second electrodes 43a and 43b measured in step S2 and the impedance IM1 between the pair of first electrodes 33a and 33b measured in step S4.

Specifically, step S5 includes step S51 and step S52.

In step S51, the control unit 102 calculates a correction coefficient C based on the impedance IM2 between the pair of second electrodes 43a and 43b measured through the reference liquid injected into the liquid receiver FW (the second channel 41) and the impedance IM1 between the pair of first electrodes 33a and 33b measured through the sample liquid injected into the liquid receiver FW (the first channel 31). In this case, the impedance IM1 is an impedance between the first electrodes 33a and 33b measured when the measurement voltage Vm1 having the specific frequency fx is applied between the first electrodes 33a and 33b. Similarly, the impedance IM2 is an impedance between the second electrodes 43a and 43b measured when the measurement voltage Vm2 having the specific frequency fx is applied between the second electrodes 43a and 43b. The specific frequency fx of the measurement voltage Vm1 and the specific frequency fx of the measurement voltage Vm2 are the same.

The correction coefficient C is indicated by equation (1) using a voltage output efficiency VOE. The voltage output efficiency VOE is expressed by equation (2). Therefore, the voltage output efficiency VOE indicates the ratio of "the voltage value of the voltage actually applied to the sample liquid when the control voltage Vcn having the specific voltage value Vx is applied to first electrodes 33a and 33b" to "the specific voltage value Vx that is the target value of the voltage applied to the sample liquid."

$$C = 1/VOE \ldots (1)$$

$$VOE = (IM1-IM2)/IM1 \ldots (2)$$

Next, in step S52, the control unit 102 corrects the control voltage Vcn applied between the pair of first electrodes 33a and 33b based on the correction coefficient C. Specifically, the control unit 102 corrects the control voltage Vcn having the specific voltage value Vx based on the correction coefficient C. That is, the control unit 102 corrects the specific voltage value Vx based on the correction coefficient C and calculates a correction voltage value Vy. Specifically, the control unit 102 calculates the correction voltage value Vy by equation (3). Then, the control unit 102 controls the power supply unit 101 to output the control voltage Vcn having the correction voltage value Vy and the specific frequency fx. The specific voltage value Vx indicates the target value of the voltage applied to the sample liquid.

$$Vy = C \times Vx \ldots (3)$$

Next, in step S6, the power supply unit 101 applies the corrected control voltage Vcn between the first electrodes 33a and 33b. The corrected control voltage Vcn is a control voltage having the correction voltage value Vy and the specific frequency fx.

Next, in step S7, the control unit 102 determines whether an instruction to change the frequency of the control voltage Vcn has been received.

If it is determined in step S7 that an instruction to change the frequency has been received (YES), the process advances to step S51. Then, in step S51, the control unit 102 newly calculates the correction coefficient C based on the impedances IM1 and IM2 measured with the measurement voltages Vm1 and Vm2 having the changed frequency. In this case, the control unit 102 sets the changed frequency to the specific frequency fx.

On the other hand, if it is determined in step S7 that the instruction to change the frequency has not been received (NO), the control voltage correction method ends.

As described above with reference to FIG. 6, according to the present preferred embodiment, the voltage controller 100 corrects the control voltage Vcn applied between the pair of first electrodes 33a and 33b based on the impedance IM2 between the pair of second electrodes 43a and 43b measured through the reference liquid injected into the liquid receiver FW (the second channel 41) (step S5). Therefore, in the channel chip 11, the influence of the parasitic component on the separation performance and the collection performance of the dielectric particles P1 from the other components P2 contained in the sample liquid can be reduced. The parasitic component is a parasitic component existing inside the channel chip 11.

According to the present preferred embodiment, step S51 of calculating the correction coefficient C and step S52 of correcting the control voltage Vcn are executed each time the frequency of the control voltage Vcn is changed (step S7). Therefore, the control voltage Vcn can be appropriately corrected according to the frequency for each frequency of the control voltage Vcn.

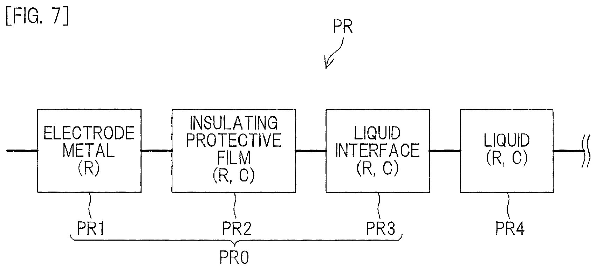

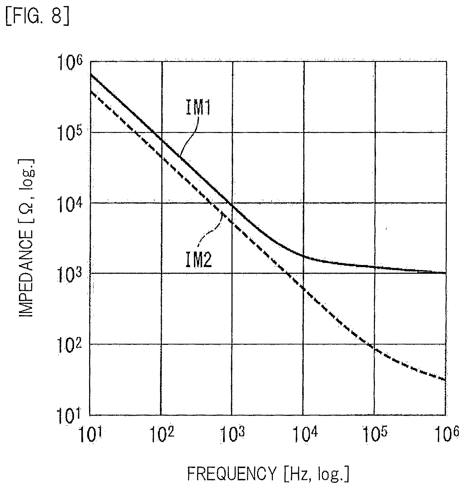

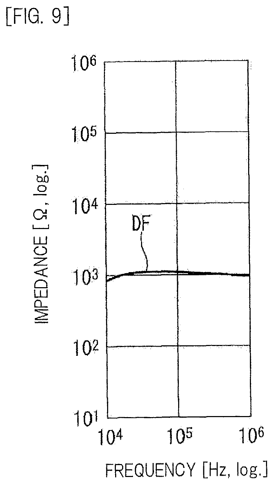

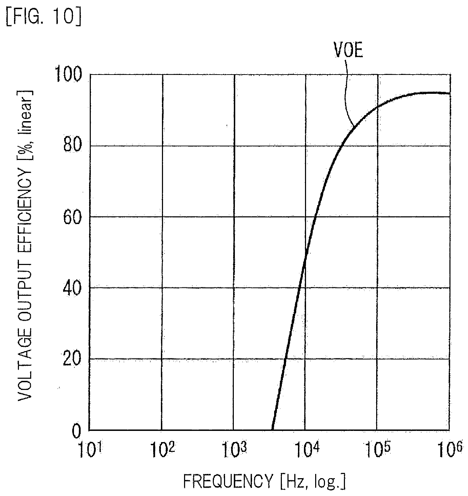

Next, the reason why the influence of the parasitic component on the separation performance and the collection performance of the dielectric particles P1 in the channel chip 11 can be reduced will be described in detail with reference to FIGS. 7 to 10. FIG. 7 is a circuit diagram of an equivalent circuit illustrating a parasitic component in the channel chip 11 (to be referred to as an "intra-device parasitic component PR" hereinafter). FIG. 8 is a graph illustrating the impedance IM1 measured through the sample liquid and the impedance IM2 measured through the reference liquid. FIG. 9 is a graph illustrating a difference value DF between the impedance IM1 and the impedance IM2 in the frequency range of $10^4$ Hz or more and $10^6$ Hz or less. FIGS. 8 and 9 illustrate a double logarithmic graph (Log-Log graph), where the abscissa represents the frequency [Hz] of the measurement voltages Vm1 and Vm2, and the ordinate represents the impedance [Ω]. FIG. 10 is a graph illustrating the frequency dependence of the voltage output efficiency VOE. Referring to FIG. 10, the abscissa represents the frequency [Hz] of the measurement voltages Vm1 and Vm2, and the ordinate represents the voltage output efficiency VOE [%]. Referring to FIG. 10, the voltage output efficiency VOE is indicated in percentage [%] for convenience in order to facilitate understanding.

First, the intra-device parasitic component PR will be described with reference to FIG. 7. As illustrated in FIG. 7, the intra-device parasitic component PR includes a parasitic component PR1 of the electrode metal, a parasitic component PR2 of the insulating protective film 23, a parasitic component PR3 of the liquid interface, and a parasitic component PR4 of the liquid. Parasitic component PR1 of the electrode metal indicates a parasitic resistance R of the first electrodes 33a and 33b or the parasitic resistance R of the second electrodes 43a and 43b. The parasitic component PR2 of the insulating protective film 23 indicates the parasitic resistance R and a parasitic capacitance C of the insulating protective film 23. The parasitic component PR3 of the liquid interface indicates the parasitic resistance R and the parasitic capacitance C at the interface between the first electrodes 33a and 33b and the sample liquid or the parasitic resistance R and the parasitic capacitance C at the interface between the second electrodes 43a and 43b and the reference liquid. The parasitic component PR4 of the liquid indicates the parasitic resistance R and the parasitic capacitance C of the sample liquid or the parasitic resistance R and the parasitic capacitance C of the reference liquid.

In this preferred embodiment, the reference liquid is used to extract a device basic parasitic component PR0. The device basic parasitic component PR0 is parasitic components PR1, PR2, and PR3 other than the parasitic component PR4 of the liquid in the intra-device parasitic component PR.

Specifically, the electric resistivity (electric resistance) of the reference liquid is smaller than the electric resistivity (electric resistance) of the second electrodes 43a and 43b. In other words, the conductivity of the reference liquid is larger than the conductivity of the second electrodes 43a and 43b. Therefore, as illustrated in FIG. 8, the impedance IM2 between the second electrodes 43a and 43b measured by the measurement device 200 through the reference liquid indicates the device basic parasitic component PR0. As described above, in the present preferred embodiment, the device basic parasitic component PR0 of the channel chip 11 can be easily extracted. The electric resistance of the reference liquid is, for example, $10^3$ $\Omega$ or less in a range where the frequency of the measurement voltage Vm2 is $10^4$ Hz or more and $10^6$ Hz or less. The electric resistance of the reference liquid is, for example, $10^2$ $\Omega$ or less in a range where the frequency of the measurement voltage Vm2 is $10^5$ Hz or more and $10^6$ Hz or less.

On the other hand, as illustrated in FIG. 8, the impedance IM1 between the first electrodes 33a and 33b measured by the voltage controller 100 through the sample liquid indicates the intra-device parasitic component PR and includes the device basic parasitic component PR0. In the example of FIG. 8, when the frequency of the measurement voltage Vm1 is about $10^4$ Hz or more, the impedance IM1 becomes substantially constant. Therefore, the impedance IM1 when the frequency is about $10^4$ Hz or more mainly indicates the electric resistance of the sample liquid. This is because the reactance, which is the imaginary part of the impedance, has frequency dependence, but the resistance, which is the real part of the impedance, does not have frequency dependence.

Therefore, as illustrated in FIG. 9, in the frequency range of $10^4$ Hz or more and $10^6$ Hz or less, the difference value DF (= IM1-IM2) obtained by subtracting the impedance IM2 from the impedance IM1 indicates the "parasitic resistance (electric resistance) of the sample liquid" which is the parasitic component PR4 (FIG. 7) of the liquid. In the example of FIG. 8, the difference value DF indicating the parasitic resistance (electric resistance) of the sample liquid is about 1 k$\Omega$.

Using the difference value DF (= IM1-IM2), the voltage output efficiency VOE can be calculated by equation (2) given above. As can be understood from FIG. 10, when the frequency is about 10 kHz or more, the voltage output efficiency VOE is 50% or more. Even when the frequency is about 10 kHz or more, the voltage output efficiency VOE depends on the frequency.

The voltage output efficiency VOE indicates the ratio of "the voltage value of the voltage actually applied to the sample liquid when the control voltage Vcn having the specific voltage value Vx is applied to the first electrodes 33a and 33b" to "the specific voltage value Vx that is the target value of the voltage applied to the sample liquid." Since the device basic parasitic component PR0 is present in the channel chip 11, the voltage output efficiency VOE does not become 100%. That is, since the control voltage Vcn having the specific voltage value Vx is attenuated by the device basic parasitic component PR0, the voltage output efficiency VOE does not become 100%.

Therefore, the voltage value of the control voltage Vcn is corrected by adding the voltage value attenuated by the device basic parasitic component PR0 to the specific voltage value Vx. As a result, the voltage value of the voltage actually applied to the sample liquid can be set to the specific voltage value Vx that is the target value. That is, in the present preferred embodiment, it is possible to reduce the influence of the device basic parasitic component PR0 on the separation performance and the collection performance of the dielectric particles P1 from the other components P2 contained in the sample liquid in the channel chip 11.

Specifically, the control unit 102 calculates the correction coefficient C from the voltage output efficiency VOE and multiplies the specific voltage value Vx by the correction coefficient C to calculate the correction voltage value Vy as expressed by equations (1) and (3) given above. Since the correction coefficient C is the reciprocal of the voltage output efficiency VOE, by multiplying the specific voltage value Vx by the correction coefficient C, it is possible to obtain the correction voltage value Vy obtained by adding the voltage value attenuated by the device basic parasitic component PR0 to the specific voltage value Vx. The correction coefficient C is a real number larger than 1. Then, the control unit 102 controls the power supply unit 101 to apply the control voltage Vcn having the correction voltage value Vy to the first electrodes 33a and 33b. As a result, the power supply unit 101 applies the control voltage Vcn having the correction voltage value Vy to the first electrodes 33a and 33b. According to the present preferred embodiment, the control voltage Vcn can be easily corrected by measuring the impedances IM1 and IM2 and calculating the correction coefficient C.

Here, when the plurality of channel chips 11 are manufactured, manufacturing variation may occur within the tolerance among the plurality of channel chips 11. For example, when the plurality of channel chips 11 are manufactured, the thickness and structure of the insulating protective film 23 may vary within the tolerance among the plurality of channel chips 11. In particular, parasitic component PR2 (FIG. 7) of the insulating protective film 23 is a high reactance component. Therefore, in particular, when variation in the insulating protective film 23 occurs among the plurality of channel chips 11, the difference voltage between the control voltage Vcn applied to the first electrodes 33a and 33b and the voltage actually applied to the sample liquid can also vary. As a result, without correction, the separation performance and the collection performance of the dielectric particles P1 to be separated may also vary among the plurality of channel chips 11.

Therefore, in the present preferred embodiment, the voltage value of the control voltage Vcn is corrected by adding the voltage value attenuated by the device basic parasitic component PR0 including the parasitic component PR2 of the insulating protective film 23 to the specific voltage value Vx. That is, the influence of the device basic parasitic component PR0 is reduced by calculating the correction coefficient C by equations (1) to (3) and correcting the control voltage Vcn by the correction coefficient C. As a result, even when there is manufacturing variation of the insulating protective film 23 and the like among the plurality of channel chips 11, variation in the performance of the plurality of channel chips 11 can be suppressed.

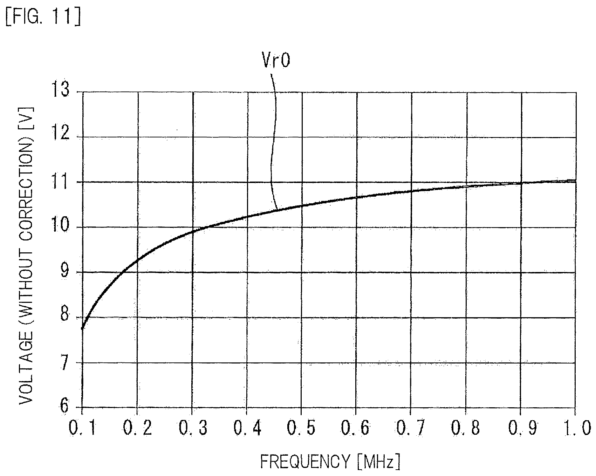

Next, correction processing of the control voltage Vcn will be described with specific examples with reference to FIGS. 11 to 13. In the description of FIGS. 11 to 13, as an example, the specific voltage value Vx, which is the target value of the voltage applied to the sample liquid, is set to "12 V."

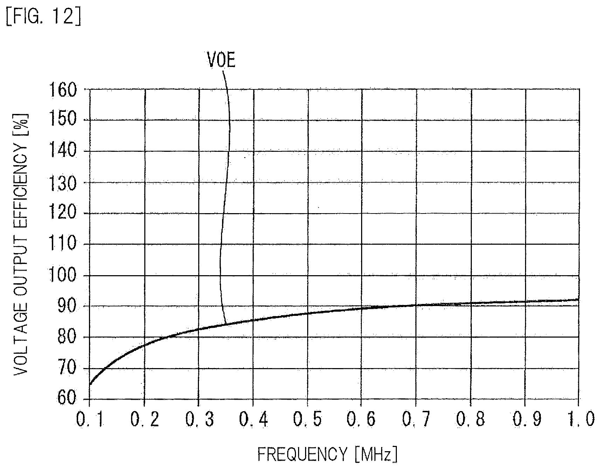

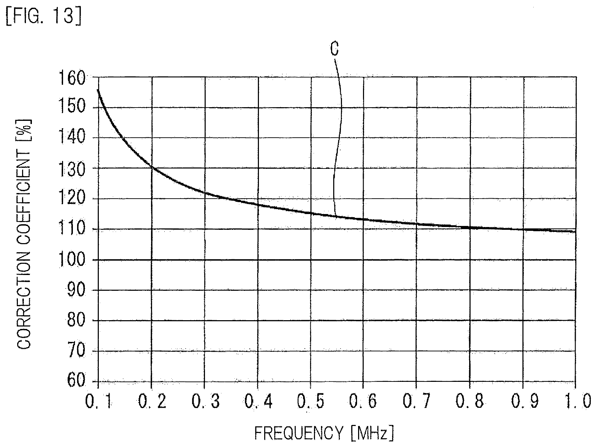

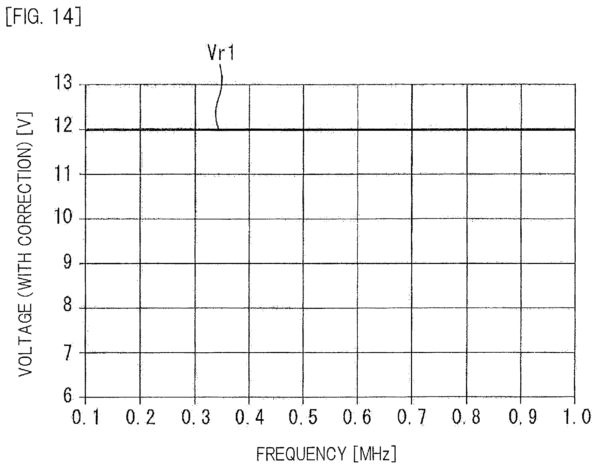

FIG. 11 is a graph showing a voltage value Vr0 of the voltage actually applied to the sample liquid when the control voltage Vcn having the specific voltage value Vx is applied to the first electrodes 33a and 33b. The abscissa represents the frequency [MHz] of the control voltage Vcn, and the ordinate represents the voltage [V]. FIG. 12 is a graph illustrating the voltage output efficiency VOE corresponding to the voltage value Vr0 in FIG. 11. The abscissa represents the frequency [MHz] of the control voltage Vcn, and the ordinate represents the voltage output efficiency VOE [%]. Referring to FIG. 12, the voltage output efficiency VOE is indicated in percentage [%] for convenience in order to facilitate understanding. FIG. 13 is a graph illustrating the correction coefficient C. Referring to FIG. 13, the correction coefficient C is indicated in percentage [%] for convenience in order to facilitate understanding. As indicated by equation (1) given above, the correction coefficient C is indicated by the reciprocal of the voltage output efficiency VOE (1/VOE) (C = 1/VOE). The abscissa represents the frequency [MHz] of the control voltage Vcn, and the ordinate represents the correction coefficient C [%]. FIG. 14 is a graph showing a voltage value Vr1 of the voltage actually applied to the sample liquid when the corrected control voltage Vcn is applied to the first electrodes 33a and 33b. The abscissa represents the frequency [MHz] of the control voltage Vcn, and the ordinate represents the voltage [V].

As illustrated in FIG. 11, when the power supply unit 101 applies the control voltage Vcn having the specific voltage value Vx (= 12 V) to the first electrodes 33a and 33b, voltage attenuation occurs due to the influence of the device basic parasitic component PR0, and the voltage value Vr0 of the voltage actually applied to the sample liquid is smaller than the specific voltage value Vx (= 12 V). Also, the voltage value Vr0 changes according to the frequency of the control voltage Vcn.

Therefore, as illustrated in FIG. 12, the control unit 102 calculates the voltage output efficiency VOE for each frequency. The voltage output efficiency VOE takes a value corresponding to the voltage value Vr0 illustrated in FIG. 11.

Furthermore, as illustrated in FIG. 13, the control unit 102 calculates the correction coefficient C that is the reciprocal of the voltage output efficiency VOE illustrated in FIG. 12 for each frequency. Then, the control unit 102 calculates the correction voltage value Vy using the correction coefficient C and the specific voltage value Vx for each frequency according to equation (3) given above. As a result, the power supply unit 101 applies the control voltage Vcn having the correction voltage value Vy to the first electrodes 33a and 33b.

Therefore, as illustrated in FIG. 14, when the corrected control voltage Vcn is applied to the first electrodes 33a and 33b, the voltage value Vr1 of the voltage actually applied to the sample liquid becomes substantially constant without depending on the frequency. Specifically, the voltage value Vr1 of the voltage actually applied to the sample liquid substantially matches the specific voltage value Vx (= 12 V) which is a target value. In this way, the influence of the device basic parasitic component PR0 can be reduced, and the voltage of the specific voltage value Vx (voltage value Vr1), which is the target value, can be applied to the sample liquid.

Also, even when there is manufacturing variation of the insulating protective film 23 and the like among the plurality of channel chips 11, the voltage value Vr1 of the voltage actually applied to the sample liquid substantially coincides with the specific voltage value Vx (= 12 V) as the target value. As described above, by reducing the influence of the device basic parasitic component PR0 including the parasitic component PR2 and the like of the insulating protective film 23, it is possible to suppress variations in the separation performance and the collection performance of the dielectric particles P1 by the channel chip 11 among the plurality of channel chips 11.

Furthermore, as illustrated in FIG. 13, the correction coefficient C has a value corresponding to the frequency of the control voltage Vcn. Therefore, according to the present preferred embodiment, the control voltage Vcn can be appropriately corrected according to the frequency for each frequency of the control voltage Vcn. As a result, as illustrated in FIG. 14, the voltage value Vr1 of the voltage actually applied to the sample liquid over a wide frequency range can be made to substantially coincide with the specific voltage value Vx (= 12 V) which is the target value.

(First modified example)

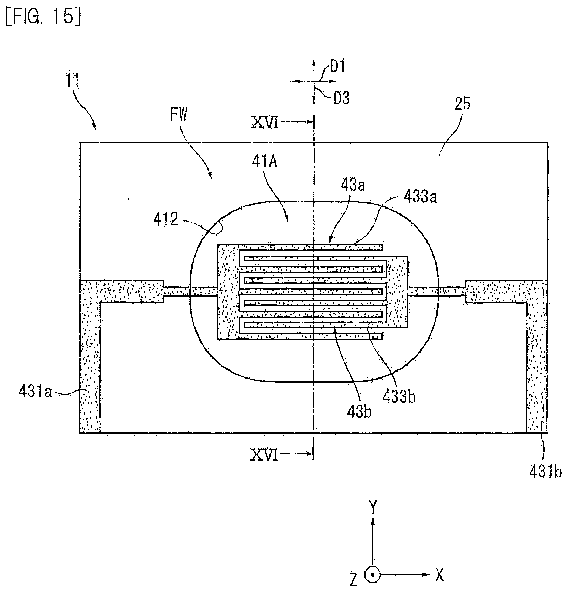

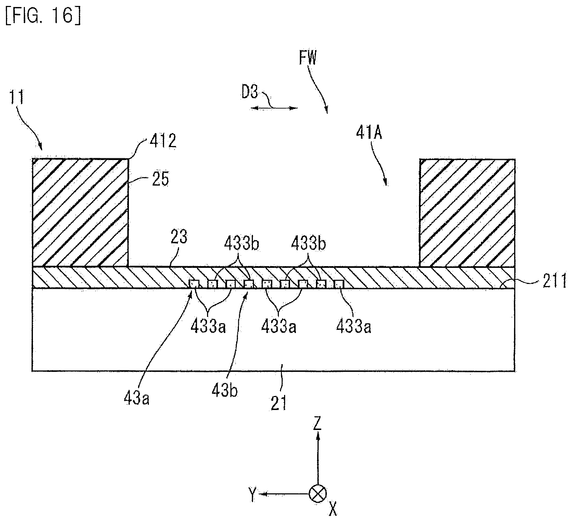

A channel chip 11 according to the first modified example of the present preferred embodiment will be described with reference to FIGS. 15 and 16. The first modified example is mainly different from the above preferred embodiment in that the channel chip 11 according to the first modified example includes a retention portion 41A instead of the second channel 41 (FIG. 1). Hereinafter, differences between the first modified example and the above preferred embodiment will be mainly described.

FIG. 15 is a plan view illustrating the retention portion 41A and the second electrodes 43a and 43b of the channel chip 11 according to the first modified example. FIG. 16 is a schematic cross-sectional view taken along line XVI-XVI in FIG. 15. FIGS. 15 and 16 illustrate only the periphery of the retention portion 41A and the retention portion 41A in the channel chip 11.

As illustrated in FIGS. 15 and 16, the channel chip 11 includes the retention portion 41A instead of the second channel 41 illustrated in FIG. 1. The retention portion 41A can retain the reference liquid. The retention portion 41A is separated from the first channel 31 (FIG. 1). As illustrated in FIG. 16, the retention portion 41A is disposed on a side of the principal surface 211 of the substrate 21. The retention portion 41A is formed of a through hole provided in the channel cover 25. The channel cover 25 has an opening portion 412 for injecting the reference liquid into the retention portion 41A. The retention portion 41A is not an elongated tubular channel surrounded by walls on four sides as in the second channel 41 (FIG. 3) but is configured as a recessed hole opening upward.

As illustrated in FIGS. 15 and 16, the channel chip 11 includes the pair of second electrodes 43a and 43b. The pair of second electrodes 43a and 43b are separated from the first channel 31 (FIG. 1) and the pair of first electrodes 33a and 33b (FIG. 1) and are disposed between the principal surface 211 of the substrate 21 and the retention portion 41A. The second electrodes 43a and 43b are covered with the insulating protective film 23.

The second electrode 43a has the plurality of second line portions 433a arranged in a comb shape. The second electrode 43b has the plurality of second line portions 433b arranged in a comb shape. The plurality of second line portions 433a and the plurality of second line portions 433b extend along the direction D1. The plurality of second line portions 433a are arranged at equal intervals in the direction D3. The plurality of second line portions 433b are arranged at equal intervals in the direction D3. The second line portions 433a and the second line portions 433b are alternately arranged at intervals in the direction D3.

In the case of the channel chip 11 according to the first modified example, the measurement device 200 measures the impedance IM2 between the second electrodes 43a and 43b in a state where the reference liquid is injected into the retention portion 41A. Then, the control voltage Vcn applied between the pair of first electrodes 33a and 33b is corrected based on the impedance IM2 between the pair of second electrodes 43a and 43b measured through the reference liquid injected into the retention portion 41A. Therefore, according to the first modified example, similar to the above preferred embodiment, in the channel chip 11, the influence of the parasitic component on the separation performance and the collection performance of the dielectric particles P1 from the other components P2 contained in the sample liquid can be reduced. Also, it is possible to suppress variations in the separation performance and the collection performance of the dielectric particles P1 by the channel chip 11 among the plurality of channel chips 11.

Specifically, the control voltage Vcn applied between the pair of first electrodes 33a and 33b is corrected based on the impedance IM2 between the pair of second electrodes 43a and 43b measured through the reference liquid injected into the retention portion 41A and the impedance IM1 between the pair of first electrodes 33a and 33b measured through the sample liquid injected into the first channel 31.

According to the first modified example, the first channel 31 into which the sample liquid is injected and the retention portion 41A into which the reference liquid is injected are separated from each other. Then, the impedance IM1 of the sample liquid and the impedance IM2 of the reference liquid are separately measured. Therefore, the first channel 31 can be prevented from being contaminated with the reference liquid, and the retention portion 41A can be prevented from being contaminated with the sample liquid. Also, the measurement of the impedance IM1 and the measurement of the impedance IM2 can be executed in parallel. As a result, the throughput of the separation and collection processing of the dielectric particles P1 can be improved.

(Second modified example)

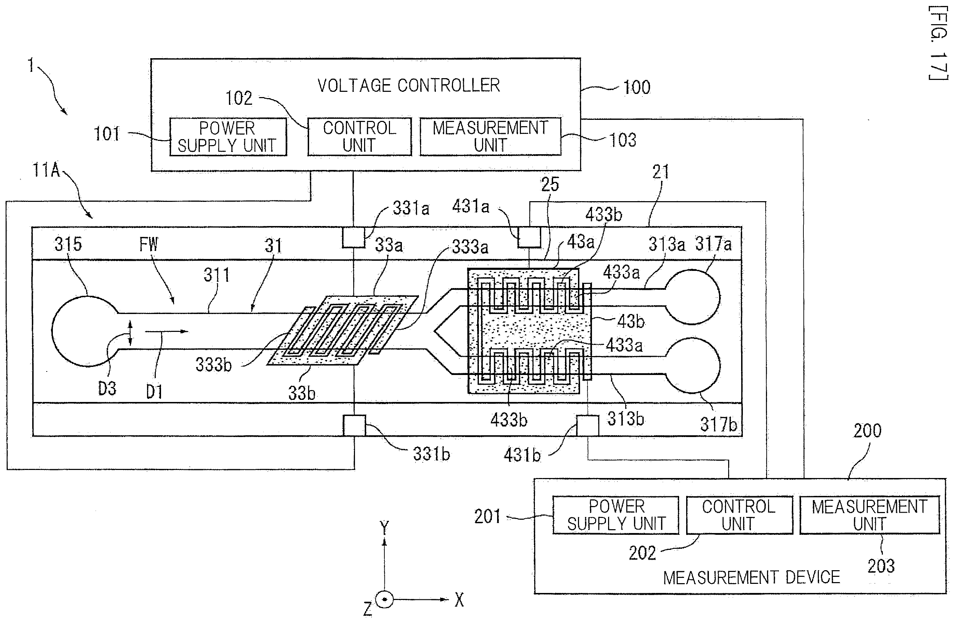

A dielectrophoresis apparatus 1 according to the second modified example of the present preferred embodiment will be described with reference to FIG. 17. The second modified example is mainly different from the above preferred embodiment in that a channel chip 11A according to the second modified example includes the first electrodes 33a and 33b and the second electrodes 43a and 43b in the first channel 31. Hereinafter, differences between the second modified example and the above preferred embodiment will be mainly described.

FIG. 17 is a plan view illustrating the dielectrophoresis apparatus 1 according to the second modified example. As illustrated in FIG. 17, the liquid receiver FW includes the first channel 31.

The pair of first electrodes 33a and 33b can cause a dielectrophoretic force to act on the dielectric particles P1 contained in the sample liquid injected into the first channel 31 in a state where the reference liquid is not injected into the first channel 31. That is, the voltage controller 100 (the power supply unit 101) applies the control voltage Vcn to the first electrodes 33a and 33b in a state where the reference liquid is not injected into the first channel 31 and the sample liquid is injected into the first channel 31.

The voltage controller 100 (the power supply unit 101) can also apply the measurement voltage Vm1 to the first electrodes 33a and 33b in a state where the reference liquid is not injected into the first channel 31 and the sample liquid is injected into the first channel 31. In this case, the voltage controller 100 (the measurement unit 103) measures the impedance IM1 between the pair of first electrodes 33a and 33b in a state where the sample liquid is injected into the first channel 31.

On the other hand, the pair of second electrodes 43a and 43b are disposed at positions different from positions of the pair of first electrodes 33a and 33b between the principal surface 211 (FIG. 3) of the substrate 21 and the first channel 31 (FIG. 3).

The measurement device 200 (the power supply unit 201) applies the measurement voltage Vm2 to the second electrodes 43a and 43b in a state where the sample liquid is not injected into the first channel 31 and the reference liquid is injected into the first channel 31. Then, the measurement device 200 (the measurement unit 203) measures the impedance IM2 between the pair of second electrodes 43a and 43b in a state where the reference liquid is injected into the first channel 31.

The control voltage Vcn applied between the pair of first electrodes 33a and 33b is corrected based on the impedance IM2 between the pair of second electrodes 43a and 43b measured through the reference liquid injected into the first channel 31 in a state where the sample liquid is not injected into the first channel 31. Therefore, according to the second modified example, similar to the above preferred embodiment, in the channel chip 11A, the influence of the parasitic component on the separation performance and the collection performance of the dielectric particles P1 from the other components P2 contained in the sample liquid can be reduced. Also, it is possible to suppress variations in the separation performance and the collection performance of the dielectric particles P1 by the channel chip 11A among the plurality of channel chips 11A.

Specifically, the control voltage Vcn applied between the pair of first electrodes 33a and 33b is corrected based on the impedance IM2 between the pair of second electrodes 43a and 43b measured through the reference liquid injected into the first channel 31 and the impedance IM1 between the pair of first electrodes 33a and 33b measured through the sample liquid injected into the first channel 31.

Successively, the second electrodes 43a and 43b will be described with reference to FIG. 17. The second electrodes 43a and 43b are separated from the first electrodes 33a and 33b in the direction D1. The second electrodes 43a and 43b are covered with the insulating protective film 23 (FIG. 3).

The second electrode 43a has the plurality of second line portions 433a arranged in a comb shape. The second electrode 43b has the plurality of second line portions 433b arranged in a comb shape. The plurality of second line portions 433a and the plurality of second line portions 433b extend along the direction D3. The second line portions 433a and the second line portions 433b are alternately arranged at intervals in the direction D1.

Some of the plurality of second line portions 433a are substantially orthogonal to the collection channel 313a in plan view and are arranged at equal intervals in the direction D1. Some of the plurality of second line portions 433b are substantially orthogonal to the collection channel 313a in plan view and are arranged at equal intervals in the direction D1.

Other parts of the plurality of second line portions 433a are substantially orthogonal to the collection channel 313b in plan view and are arranged at equal intervals in the direction D1. Some other parts of the plurality of second line portions 433b are substantially orthogonal to the collection channel 313b in plan view, and are arranged at equal intervals in the direction D1.

According to the second modified example, the first electrodes 33a and 33b and the second electrodes 43a and 43b are arranged corresponding to the first channel 31. Therefore, as compared with a case where the second channel 41 or the retention portion 41A is separately provided, the channel chip 11A can be downsized.

The preferred embodiments of the present invention have been described above with reference to the accompanying drawings. However, the present invention is not limited to the above preferred embodiment and can be implemented in various modes without departing from the gist thereof. Also, the plurality of constituent elements disclosed in the above preferred embodiment can be modified appropriately. For example, one constituent element among all constituent elements illustrated in one preferred embodiment may be added to the constituent elements of another preferred embodiment, or some constituent elements among all constituent elements illustrated in one preferred embodiment may be deleted from the preferred embodiment.

Also, the drawings schematically show the respective constituent elements mainly in order to facilitate understanding of the present invention, and the thickness, the length, the number, the interval, etc., of each constituent element shown in the figures may be different from the actual ones for convenience in creating the drawings. Also, the configuration of each constituent element illustrated in the above preferred embodiment are merely examples, and are not particularly limited, and various modifications can be made without substantially departing from the effects of the present invention.

(1) In the present preferred embodiment, the first modified example, and the second modified example described with reference to FIGS. 1, 15, and 17, the dielectrophoresis apparatus 1 suffices to include any one of the voltage controller 100 and the measurement device 200. In a case where the dielectrophoresis apparatus 1 includes the voltage controller 100 but does not include the measurement device 200, the voltage controller 100 is connected to the second electrode pad portions 431a and 431b to cause the voltage controller 100 to measure the impedance IM2 between the second electrodes 43a and 43b. When the dielectrophoresis apparatus 1 includes the measurement device 200 but does not include the voltage controller 100, the measurement device 200 is connected to the first electrode pad portions 331a and 331b to cause the measurement device 200 to measure the impedance IM1 between the first electrodes 33a and 33b. Also, the control voltage Vcn is applied between the first electrodes 33a and 33b by the measurement device 200.

(2) In the present preferred embodiment and the first modified example described with reference to FIGS. 1 and 15, when an electrode unit is configured by the pair of second electrodes 43a and 43b as one set, the channel chip 11 may include a plurality of electrode units. In this case, the plurality of electrode units are arranged at intervals in the direction D2. Then, the correction coefficient C may be calculated based on the average value of the plurality of impedances IM2 measured by the plurality of electrode units.

(3) In the second modified example described with reference to FIG. 17, the second electrodes 43a and 43b are disposed downstream of the first electrodes 33a and 33b in the direction D1. However, the second electrodes 43a and 43b may be disposed upstream of the first electrodes 33a and 33b in the direction D1.

Also, the second electrodes 43a and 43b may be arranged corresponding to any one of the collection channel 313a and the collection channel 313b.

(4) In the control voltage correction method described with reference to FIG. 6, steps S3 and S4 may be executed before steps S1 and S2.

Industrial Applicability

The present invention relates to a channel chip, a dielectrophoresis apparatus, and a control voltage correction method and has industrial applicability. Reference Signs List

| 11, 11A | channel chip |
| 21 | substrate |
| 31 | first channel |
| 41 | second channel |
| 41A | retention portion |
| 33a, 33b | first electrode |
| 43a, 43b | second electrode |
| 100 | voltage controller |
| FW | liquid receiver |

## Claims

1. A channel chip comprising:

   a substrate;
   a liquid receiver which is disposed on one principal surface side of the substrate and into which a sample liquid or a reference liquid can be injected;
   a pair of first electrodes disposed between the principal surface of the substrate and the liquid receiver and can cause a dielectrophoretic force to act on dielectric particles contained in the sample liquid injected into the liquid receiver when a control voltage is applied; and
   a pair of second electrodes disposed at positions different from positions of the pair of first electrodes between the principal surface of the substrate and the liquid receiver; wherein
   the control voltage applied between the pair of first electrodes is corrected based on an impedance between the pair of second electrodes measured through the reference liquid injected into the liquid receiver.

2. The channel chip according to claim 1, wherein an electric resistivity of the reference liquid is smaller than an electric resistivity of the second electrode.

3. The channel chip according to claim 1 or 2, wherein

the control voltage applied between the pair of first electrodes is corrected based on a correction coefficient, and
the correction coefficient is calculated based on the impedance between the pair of second electrodes measured through the reference liquid injected into the liquid receiver and an impedance between the pair of first electrodes measured through the sample liquid injected into the liquid receiver.

4. The channel chip according to claim 3, wherein

the control voltage is an AC voltage, and
the correction coefficient has a value corresponding to a frequency of the control voltage.

5. The channel chip according to claim 1 or 2, wherein

the liquid receiver includes a first channel which is disposed on the principal surface side of the substrate and into which the sample liquid can be injected, and a second channel which is separated from the first channel and disposed on the principal surface side of the substrate and into which the reference liquid can be injected,
the pair of first electrodes are disposed between the principal surface of the substrate and the first channel,
the pair of second electrodes are separated from the first channel and the pair of first electrodes and disposed between the principal surface of the substrate and the second channel, and
the control voltage applied between the pair of first electrodes is corrected based on the impedance between the pair of second electrodes measured through the reference liquid injected into the second channel.

6. The channel chip according to claim 1 or 2, wherein

the liquid receiver includes a first channel which is disposed on the principal surface side of the substrate and into which the sample liquid can be injected,
a retention portion which is separated from the first channel and disposed on the principal surface side of the substrate and into which the reference liquid can be injected,
the pair of first electrodes are disposed between the principal surface of the substrate and the first channel,
the pair of second electrodes are separated from the first channel and the pair of first electrodes and disposed between the principal surface of the substrate and the retention portion, and

the control voltage applied between the pair of first electrodes is corrected based on the impedance between the pair of second electrodes measured through the reference liquid injected into the retention portion.

7. The channel chip according to claim 1 or 2, wherein

the liquid receiver includes a first channel,
the pair of first electrodes are disposed between the principal surface of the substrate and the first channel and can cause the dielectrophoretic force to act on the dielectric particles contained in the sample liquid injected into the first channel when the control voltage is applied in a state where the reference liquid is not injected into the first channel,
the pair of second electrodes are disposed at positions different from positions of the pair of first electrodes between the principal surface of the substrate and the first channel, and
the control voltage applied between the pair of first electrodes is corrected based on the impedance between the pair of second electrodes measured through the reference liquid injected into the first channel in a state where the sample liquid is not injected into the first channel.

8. A dielectrophoresis apparatus comprising:

a channel chip; and
a voltage controller; wherein
the channel chip includes
a substrate,
a liquid receiver which is disposed on one principal surface side of the substrate and into which a sample liquid or a reference liquid can be injected,
a pair of first electrodes disposed between the principal surface of the substrate and the liquid receiver and can cause a dielectrophoretic force to act on dielectric particles contained in the sample liquid injected into the liquid receiver when a control voltage is applied, and
a pair of second electrodes disposed at positions different from positions of the pair of first electrodes between the principal surface of the substrate and the liquid receiver; wherein
the voltage controller corrects the control voltage applied between the pair of first electrodes based on an impedance between the pair of second electrodes measured through the reference liquid injected into the liquid receiver.

9. A control voltage correction method using a channel chip, the channel chip including

a substrate,

a liquid receiver which is disposed on one principal surface side of the substrate and into which a sample liquid or a reference liquid can be injected,

a pair of first electrodes disposed between the principal surface of the substrate and the liquid receiver and can cause a dielectrophoretic force to act on dielectric particles contained in the sample liquid injected into the liquid receiver when a control voltage is applied, and

a pair of second electrodes disposed at positions different from positions of the pair of first electrodes between the principal surface of the substrate and the liquid receiver,

the control voltage correction method comprising:

a step of measuring an impedance between the pair of second electrodes through the reference liquid injected into the liquid receiver; and

a step of correcting the control voltage applied between the pair of first electrodes based on the impedance between the pair of second electrodes.

10. The control voltage correction method according to claim 9, wherein an electric resistivity of the reference liquid is smaller than an electric resistivity of the second electrode.

11. The control voltage correction method according to claim 9 or 10, further comprising a step of measuring an impedance between the pair of first electrodes through the sample liquid injected into the liquid receiver,

wherein the step of correcting the control voltage includes

a step of calculating a correction coefficient based on the impedance between the pair of second electrodes measured through the reference liquid injected into the liquid receiver and the impedance between the pair of first electrodes measured through the sample liquid injected into the liquid receiver, and

a step of correcting the control voltage applied between the pair of first electrodes based on the correction coefficient.

12. The control voltage correction method according to claim 11, wherein

the control voltage is an AC voltage, and the step of calculating the correction coefficient and the step of correcting the control voltage are executed each time a frequency of the control voltage is changed.

[FIG. 1]

EP 4 563 229 A1

[FIG. 2]

[FIG. 3]

[FIG. 4]

[FIG. 5]

[FIG. 6]

```
            ┌─────────────┐
            │    START    │
            └──────┬──────┘
                   │
                   ▼
    ┌──────────────────────────────┐
    │    INJECT REFERENCE LIQUID    │──── S1
    └──────────────┬───────────────┘
                   │
                   ▼
    ┌──────────────────────────────┐
    │   MEASURE IMPEDANCE THROUGH   │──── S2
    │       REFERENCE LIQUID        │
    └──────────────┬───────────────┘
                   │
                   ▼
    ┌──────────────────────────────┐
    │     INJECT SAMPLE LIQUID      │──── S3
    └──────────────┬───────────────┘
                   │
                   ▼
    ┌──────────────────────────────┐
    │   MEASURE IMPEDANCE THROUGH   │──── S4
    │        SAMPLE LIQUID          │
    └──────────────┬───────────────┘
                   │
    ┌ ─ ─ ─ ─ ─ ─ ─│─ ─ ─ ─ ─ ─ ─ ─ ┐ ···· S5
                   ▼
    │ ┌──────────────────────────────┐ │
      │  CALCULATE CORRECTION COEFFICIENT│
    │ │  BASED ON IMPEDANCE MEASURED  │ │── S51
      │ THROUGH REFERENCE LIQUID AND  │
    │ │  IMPEDANCE MEASURED THROUGH   │ │
      │        SAMPLE LIQUID          │
    │ └──────────────┬───────────────┘ │
                     ▼
    │ ┌──────────────────────────────┐ │
      │  CORRECT CONTROL VOLTAGE BASED│──── S52
    │ │     ON CORRECTION COEFFICIENT │ │
      └──────────────┬───────────────┘
    └ ─ ─ ─ ─ ─ ─ ─ ─│─ ─ ─ ─ ─ ─ ─ ─ ┘
                     ▼
      ┌──────────────────────────────┐
      │   APPLY CORRECTED CONTROL VOLTAGE │──── S6
      └──────────────┬───────────────┘
                     ▼                    S7
             ◇──────────────────◇
       YES  ◇  IS FREQUENCY CHANGED ◇
    ◄────────◇         ?          ◇
             ◇──────────────────◇
                     │ NO
                     ▼
              ┌─────────────┐
              │     END     │
              └─────────────┘
```

[FIG. 7]

PR

| ELECTRODE METAL (R) | INSULATING PROTECTIVE FILM (R, C) | LIQUID INTERFACE (R, C) | LIQUID (R, C) |
|---|---|---|---|
| PR1 | PR2 | PR3 | PR4 |

PR0

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

## INTERNATIONAL SEARCH REPORT

International application No.

**PCT/JP2023/019097**

| A. | CLASSIFICATION OF SUBJECT MATTER |
|---|---|

*B03C 5/02*(2006.01)i; *C12M 1/00*(2006.01)i; *G01N 37/00*(2006.01)i
FI:    B03C5/02; C12M1/00 A; G01N37/00 101

According to International Patent Classification (IPC) or to both national classification and IPC

| B. | FIELDS SEARCHED |
|---|---|

Minimum documentation searched (classification system followed by classification symbols)

B03C3/00-11/00, C12M1/00-3/10, G01N27/00-27/92, G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2012-098063 A (SONY CORP.) 24 May 2012 (2012-05-24) | 1-12 |
| A | WO 2019/017094 A1 (SHARP KK) 24 January 2019 (2019-01-24) | 1-12 |
| A | JP 2012-141263 A (SHARP KK) 26 July 2012 (2012-07-26) | 1-12 |
| A | JP 59-182354 A (SHIMAZU SEISAKUSHO KK) 17 October 1984 (1984-10-17) | 1-12 |
| A | US 2018/0372667 A1 (PROBIUSDX) 27 December 2018 (2018-12-27) | 1-12 |

☐ Further documents are listed in the continuation of Box C.    ☑ See patent family annex.

| | |
|---|---|
| \*    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **20 July 2023** | **01 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/019097**

| Patent document cited in search report | | | Publication date (day/month/year) | Patent family member(s) | | | Publication date (day/month/year) |
|---|---|---|---|---|---|---|---|
| JP | 2012-098063 | A | 24 May 2012 | US | 2012/0103817 | A1 | |
| | | | | EP | 2447703 | A2 | |
| | | | | CN | 102539484 | A | |
| WO | 2019/017094 | A1 | 24 January 2019 | US | 2020/0166472 | A1 | |
| JP | 2012-141263 | A | 26 July 2012 | (Family: none) | | | |
| JP | 59-182354 | A | 17 October 1984 | (Family: none) | | | |
| US | 2018/0372667 | A1 | 27 December 2018 | CN | 110785656 | A | |
| | | | | EP | 3642603 | A1 | |
| | | | | WO | 2018/237348 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017134020 A